# EUROPÄISCHE PATENTSCHRIFT

(11) **EP 1 654 214 B1**
(45) Veröffentlichungstag und Bekanntmachung des Hinweises auf die Patenterteilung: **28.03.2007**
(21) Anmeldenummer: 04740778.8
(22) Anmeldetag: 08.07.2004
(51) Int. Cl.: C07C 209/16, C07C 211/10, C07C 211/14

(54) **VERFAHREN ZUR HERSTELLUNG VON ETHYLENAMINEN**
METHOD FOR PRODUCING ETHYLENEAMINES
PROCEDE DE PRODUCTION D'AMINES D'ETHYLENE

(30) Priorität: 01.08.2003 DE 10335991
(43) Veröffentlichungstag der Anmeldung: 10.05.2006
(73) Patentinhaber: BASF Aktiengesellschaft, 67056 Ludwigshafen (DE)
(72) Erfinder: FRAUENKRON, Matthias, 67251 Freinsheim (DE); EVERS, Holger, 68159 Mannheim (DE); MELDER, Johann-Peter, 67459 Böhl-Iggelheim (DE); RÖTTGER, Roderich, 68165 Mannheim (DE); SIEGERT, Markus, 69126 Heidelberg (DE); GERLACH, Till, 67071 Ludwigshafen (DE); NOUWEN, Jan, 2960 Brecht (BE); KRUG, Thomas, 67550 Worms (DE)
(86) Internationale Anmeldenummer: PCT/EP2004/007471
(87) Internationale Veröffentlichungsnummer: WO 2005/014523

(56) Entgegenhaltungen:
- EP-A- 0 197 611

## Beschreibung

Die vorliegende Erfindung betrifft ein Verfahren zur Herstellung von Ethylenaminen durch Umsetzung von Monoethanolamin (MEOA) mit Ammoniak in Gegenwart eines Katalysators in einem Reaktor (1) und Auftrennung des resultierenden Reaktionsaustrags.

Ethylenamine finden Verwendung als Lösungsmittel, Stabilisatoren, zur Synthese von Chelat-Bildnern, Kunstharzen, Arzneimitteln, Inhibitoren und grenzflächenaktiven Substanzen.

Insbesondere Diethylentriamin (Bis(2-aminoethyl)amin; DETA) findet Verwendung als Lösungsmittel für Farbstoffe und ist Ausgangsmaterial zur Herstellung von lonenaustauschern, Schädlingsbekämpfungsmitteln, Antioxidantien, Korrosionsschutzmitteln, Komplexbildnern, Textilhilfsmitteln und Absorptionsmitteln für (saure) Gase.

Zur Herstellung von Ethylenaminen, darunter DETA, sind in der Literatur zahlreiche Verfahren beschrieben.

Gemäß PEP Report No. 138, "Alkyl Amines", SRI International, 03/1981, insbesondere Seiten 7, 8, 13-16, 43-107, 113, 117, liefert die Umsetzung von Dichlorethan mit Ammoniak bei Molverhältnissen von 1 : 15 Diethylentriamin (DETA) mit einem Anteil an den gebildeten Ethylenaminen von größer 20 Gew.-%. Neben 40 Gew.-% Ethylendiamin (EDA) fallen jedoch 40 Gew.-% höhere Ethylenamine an.

Durch Aminierung von Monoethanolamin (MEOA) mit Ammoniak (vgl. z.B. den o.g. PEP Report) kann die Bildung dieser höheren Ethylenamine (d.h. Ethylenaminen mit einem Siedepunkt über dem von Triethylentetramin (TETA)) zugunsten von Ethylendiamin weitgehend zurückgedrängt werden. Als Nebenprodukte fallen jedoch bei dieser Umsetzung Aminoethylethanolamin (AEEA) und Piperazin (PIP) an. Da der Marktbedarf dieser beiden Produkte im Vergleich zu Ethylendiamin und Diethylentriamin schwankend ist, wurden mehrere Methoden entwickelt, den Anteil von Ethylendiamin und Diethylentriamin zulasten von Aminoethylethanolamin und Piperazin zu erhöhen.

In der Regel wird das erreicht, indem man Monoethanolamin an Übergangsmetallkatalysatoren (z.B. Ni-, Co-, Cu-Katalysatoren; US 4,014,933 (BASF AG)) in Gegenwart von Wasserstoff mit einem molaren Überschuss von Ammoniak (NH₃ : MEOA > 6) nur partiell (40-60 %) umsetzt.

Durch Zusatz von Wasser (US 3,766,184), Variation der Wasserstoffmenge (US 4,234,730 (Texaco)) und Kontrolle der MEOA-Belastung (US 4,647,701 (UCC)) gelingt es, den Anteil an Piperazin plus Aminoethylethanolamin an den gebildeten Ethylenaminen bei MEOA-Umsätzen von 40-60 % unter 20 Gew.-% zu halten. Bedingt durch den hohen Ammoniaküberschuss und den partiellen Umsatz von MEOA liegt der Anteil an Diethylentriamin an den gebildeten Ethylenaminen jedoch deutlich unter 20 Gew.-%.

Zur gezielten Herstellung von Diethylentriamin wird in GB-A-2,147,896 (Mitsui Toatsu) die Umsetzung von Monoethanolamin mit Ethylendiamin und Ammoniak (EDA : MEOA : NH₃ im Molverhältnis 2 : 1 : 18) in Gegenwart eines Phosphat-haltigen Katalysators beschrieben. Bei MEOA-Umsätzen von 65 % werden dabei DETA-Selektivitäten > 90 % beschrieben. Von Nachteil ist dabei, dass Ammoniak im Überschuss eingesetzt werden muss und hohe DETA-Selektivitäten nur in Gegenwart von EDA bei partiellem MEOA-Umsatz erreicht werden. Generelles Problem dieser Technologie ist außerdem die geringe Standzeit der eingesetzten Katalysatoren unter den drastischen Reaktionsbedingungen (280-350°C).

Zur Behebung dieser Schwäche wurde eine Vielzahl unterschiedlicher Phosphathaltiger Katalysatoren zum Patent angemeldet (US 4,683,335 (Texaco), US 4,612,397 (Texaco), US 4,609,761 (Texaco)). Abgesehen von der Gasphasenaminierung von Hydroxyethylpiperazin zu Triethylendiamin konnten sich diese Katalysatoren bisher technisch nicht durchsetzen.

Im Vergleich zur Phosphatkatalyse kann Ethylendiamin mit sich selbst (GB-A 1,508,460 (BASF AG); US 4,568,746 (UCC)) oder mit Monoethanolamin (US 3,714,259 (Jefferson Chemical); US 4,568,746) an Übergangsmetallkatalysatoren unter Wasserstoffatmosphäre bei erheblich milderen Bedingungen (140-210°C) umgesetzt werden.

Unter den in US 3,714,259 beschriebenen Bedingungen werden pro kg DETA ca. 0,45-0,84 kg Piperazin plus AEEA gebildet.

Höhere DETA/Piperazin-Verhältnisse werden im US-Patent 4,568,746 an Ni/Re-Katalysatoren (DETA/PIP = 5,4-8,9 bei 23-33 %igem Umsatz) bei Temperaturen > 170°C und in GB-A-1,508,460 an Ni/Co/Cu-Katalysatoren (DETA/PIP = 17-26 bei 23 %igem Umsatz) bei Temperaturen < 150°C und bevorzugten Drucken von 25 bis 45 bar erzielt.

US 5,410,086 (Burgess) beansprucht die Steuerung des DETA/Piperazin-Verhältnisses durch Einstellung der Wasserstoffkonzentration in der flüssigen Phase.

Nachteile dieser Technologien (stand alone) sind, dass dabei kein Ethylendiamin produziert wird und Ammoniak, der durch Kondensation von Ethylendiamin freigesetzt wird, als Einsatzstoff verloren geht.

DD-A-213 206 betrifft ein Verfahren zur Herstellung von Di- und Polyethylenpolyaminen durch Aminierung von Monoethanolamin an einem Hydrierkatalysator in einer Vorreaktionszone und einer hiermit verbundenen Hauptreaktionszone.

DD-A-217 507 beschreibt ein Verfahren zur Herstellung von Di- und Polyethylenpolyaminen durch Aminierung von Monoethanolamin an einem Hydrierkatalysator in zwei Reaktionsschritten, wobei das primäre Aminierungsprodukt aus dem ersten Reaktionsschritt nach Abtrennung des überschüssigen Ammoniaks zu einem sekundären Aminierungsprodukt umgesetzt wird.

In der EP-A2-197 611 (Union Carbide Corp.) wird ein Verfahren beschrieben, in dem der Anteil höherer Ethylenamine an den gebildeten Ethylenaminen durch Einsatz von zwei hintereinander geschaltenen Reaktor erhöht wird.

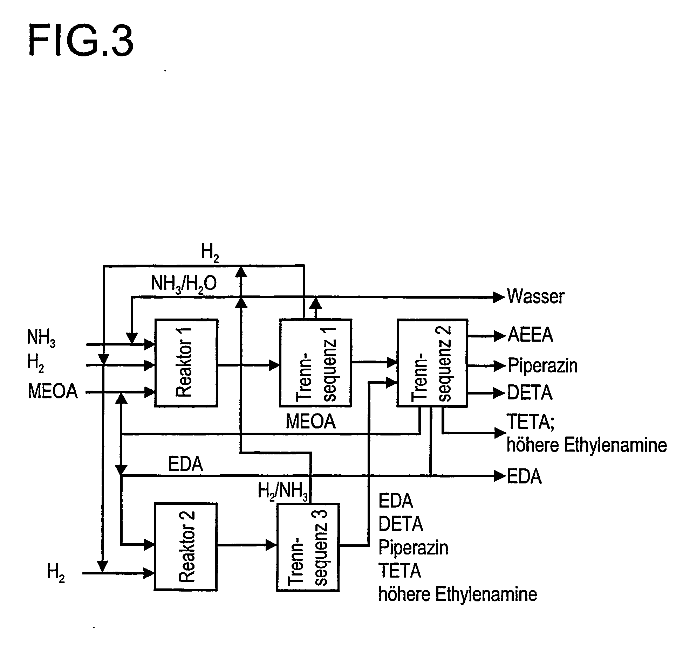

Das Verfahren ist in Abbildung 1 (Anlage 1) skizziert. Vgl. auch Fig. 3 in der EP-A-2197611.

Im ersten Reaktor erfolgt die Aminierung von MEOA mit Ammoniak an Übergangsmetallkatalysatoren (Ni, Re, Träger).

Der Reaktoraustrag wird zur Erhöhung des Anteils höherer Ethylenamine über einen zweiten Reaktor, der ebenfalls mit einem Übergangsmetallkatalysator oder mit einem Phosphatkatalysator beladen ist, geschickt.

Zur Steuerung der Produktverteilung und Erhöhung der Selektivität bezüglich der linearen Ethylenamine wird vor dem zweiten Reaktor Ethylendiamin, das aus der Aufarbeitung des Reaktionsaustrags des zweiten Reaktors stammt und auch MEOA und H₂O enthält, zugefahren.

Nachteil dieses Verfahrens ist, dass AEEA bevorzugt zu Piperazin und nicht zu DETA weiterreagiert und durch Umsetzung von EDA mit MEOA zusätzliche Mengen an AEEA gebildet werden.

Der vorliegenden Erfindung lag die Aufgabe zugrunde, ein verbessertes wirtschaftliches Verfahren zur Herstellung von Ethylenaminen, wobei es sich bei den Ethylenaminen insbesondere um Ethylendiamin (EDA), Diethylentriamin (DETA), Aminoethylethanolamin (AEEA), Piperazin (PIP) und/oder Triethylentetramin (TETA) handelt, aufzufinden, wobei der Anteil an Diethylentriamin an den gebildeten Ethylenaminen größer 20 Gew.-% beträgt und der Anteil an Piperazin plus Aminoethylethanolamin an den gebildeten Ethylenaminen je nach Bedarf auf unter 15 Gew.-% begrenzt werden kann, bei einer Gesamtausbeute bezüglich EDA, DETA, AEEA und Piperazin von größer 90%.

Demgemäß wurde ein Verfahren zur Herstellung von Ethylenaminen durch Umsetzung von Monoethanolamin (MEOA) mit Ammoniak in Gegenwart eines Katalysators in einem Reaktor (1) und Auftrennung des resultierenden Reaktionsaustrags gefunden, welches dadurch gekennzeichnet ist, dass bei der Auftrennung erhaltenes Ethylendiamin (EDA) in einem separaten Reaktor (2) in Gegenwart eines Katalysators zu Diethylentriamin (DETA) umgesetzt und der resultierende Reaktionsaustrag der Auftrennung des aus Reaktor 1 resultierenden Reaktionsaustrags zugeführt wird.

Das Verfahren lässt sich wie folgt ausführen.

Die Umsetzung von Monoethanolamin mit Ammoniak im Reaktor 1, der natürlich auch in zwei oder mehr seriell oder parallel geschaltete Reaktoren aufgeteilt sein kann, kann nach dem Fachmann bekannten Verfahren durchgeführt werden (siehe z.B. PEP Report No. 138, "Alkyl Amines", SRI International, 03/1981, Seiten 81-99, 117, und eingangs zitierte Literatur).

Die Umsetzung von Monoethanolamin mit Ammoniak wird im Reaktor (1) bevorzugt an einem Übergangsmetallkatalysator bei im allgemeinen 150-250 bar und im allgemeinen 160-210°C oder an einem Zeolithkatalysator bei im allgemeinen 1-20 bar und im allgemeinen 280-380°C durchgeführt.

Beim Reaktor 1 handelt es sich bevorzugt um einen Festbettreaktor.

Bevorzugt verwendete Übergangsmetalle im Katalysator sind Ni, Co, Cu, Ru, Re, Rh, Pd oder Pt oder eine Mischung zweier oder mehrerer dieser Metalle auf einem oxidischen Träger (z.B. Al₂O₃, TiO₂, ZrO₂, SiO₂).

Bevorzugte Zeolithkatalysatoren sind Mordenite, Faujasite und Chabazite.

Zur Erzielung einer möglichst hohen Selektivität bezüglich der linearen Amine EDA und DETA wird bei der Übergangsmetallkatalyse im allgemeinen mit einem molaren Verhältnis von Ammoniak zu Monoethanolamin von 6-20, bevorzugt 8-15, und bei Zeolithkatalyse von im allgemeinen 20-80, bevorzugt 30-50, gearbeitet.

Der MEOA-Umsatz wird im allgemeinen im Bereich zwischen 10 % und 80 %, bevorzugt 40-60 %, gehalten.

Unter den angegebenen Reaktionsbedingungen wird im bevorzugt kontinuierlichen Betrieb bei einer WHSV (weight hourly space velocity) im Bereich von 0,3-0,6 kg/(kg*h) (kg MEOA pro kg Kat. pro Stunde) eine Selektivität für EDA+DETA bezüglich umgesetztem MEOA von bevorzugt > 80 %, insbesondere 83-85 %, erreicht.

Zur Aufrechterhaltung der Katalysatoraktivität werden bei Einsatz von Metallkatalysatoren bevorzugt zusätzlich 0,05-0,5 Gew.-% (bezogen auf den Reaktorfeed M-OA+NH₃+H₂) Wasserstoff in den Reaktor 1 gefahren.

Der Reaktoraustrag wird anschließend auf bevorzugt 20-30 bar entspannt. Der dabei anfallende "Niederdruckwasserstoff" kann direkt oder nach der Abtrennung von Ammoniak über eine Gaswäsche als Feed für den Reaktor 2 (siehe unten) verwendet werden.

Der nach Abtrennung des Wasserstoffs verbleibende Reaktionsaustrag enthaltend im Wesentlichen oder bestehend aus Ammoniak, Wasser, Ethylendiamin, Piperazin, Monoethanolamin, Diethylentriamin, Aminoethylethanolamin, Triethylentetramin (TETA) und höheren Ethylenaminen (d.h. Ethylenaminen mit einem (bei gleichem Druck) höheren Siedepunkt als TETA) wird den Dampfdrucken entsprechend in die einzelnen Bestandteile aufgetrennt.

Die mehrsufige Auftrennung in die Bestandteile erfolgt bevorzugt destillativ, insbesondere durch kontinuierliche Destillation. Solche Verfahren zur Auftrennung sind dem Fachmann z.B. aus dem o.g. PEP Report No. 138 bekannt.

Die zur destillativen Reingewinnung der einzelnen Produkte, vor allem der gewünschten Ethylenamine, benötigten Destillationskolonnen können durch den Fachmann mit ihm geläufigen Methoden ausgelegt werden (z.B. Zahl der Trennstufen, Rücklaufverhältnis, etc.).

Besonders bevorzugt ist die Auftrennung des aus Reaktor 1 resultierenden Reaktionsaustrags in zwei Trennsequenzen durch mehrstufige Destillation, wobei in der ersten Trennsequenz (Trennsequenz 1) zunächst Ammoniak, Wasser und gegebenenfalls vorhandener Wasserstoff abgetrennt werden und in der zweiten Trennsequenz (Trennsequenz 2) eine Auftrennung in EDA, PIP, MEOA, DETA, AEP, HEP, AEEA, TETA und höhere Ethylenamine erfolgt.
(AEP = N-(2-Aminoethyl)-piperazin; HEP = N-(2-Hydroxyethyl)-piperazin).

Bei dieser Auftrennung des aus Reaktor 1 resultierenden Reaktionsaustrags durch unvollständige Umsetzung gegebenenfalls anfallendes Monoethanolamin wird bevorzugt in den Reaktor 1 zurückgeführt.

Das bei dieser Auftrennung anfallende Ethylendiamin (EDA) wird, gegebenenfalls je nach Bedarf nach Abzweigung einer Teilmenge in einen Lagertank, in einen separaten Reaktor (2) zur Umsetzung zu Diethylentriamin (DETA) in Gegenwart eines Katalysators gefahren.

Die Umsetzung von EDA zu DETA im Reaktor 2, der natürlich auch in zwei oder mehr seriell oder parallel geschaltete Reaktoren aufgeteilt sein kann, kann nach dem Fachmann bekannten Verfahren (siehe z.B. US 5,410,086 (Burgess) und GB-A-1,508,460 (BASF AG) und WO-A1-03/010125 (Akzo Nobel)) durchgeführt werden.

Die Umsetzung von Ethylendiamin zu Diethylentriamin erfolgt bevorzugt an einem Übergangsmetallkatalysator. Die dabei bevorzugt verwendeten Metalle sind Ni, Co, Cu, Ru, Re, Rh, Pd oder Pt oder eine Mischung zweier oder mehrerer dieser Metalle auf einem oxidischen Träger (z.B. Al₂O₃, TiO₂, ZrO₂, SiO₂).

Alternativ zu den Übergangsmetallkatalysatoren können für diese Umsetzung auch formselektive Zeolithkatalystoren oder Phosphatkatalysatoren verwendet werden.

Die Umsetzung an Übergangsmetallkatalysatoren erfolgt im allgemeinen bei einem Druck von 1-200 bar, bevorzugt bei 1-30 bar, und im allgemeinen im Temperaturbereich von 130-170°C, bevorzugt bei 140-160°C.

In einer Ausführungsform kann der Reaktor 2 auch mit einem Gemisch aus MEOA und EDA betrieben werden, wobei EDA im molaren Überschuss, z.B. im molaren EDA : MEOA - Verhältnis > 5, eingesetzt wird. Bevorzugt wird jedoch nur EDA eingesetzt, da hier im Vergleich zur EP-A2-197 611 die Bildung zusätzlicher Mengen AEEA im Reaktor 2 vollständig unterdrückt werden kann.

Zur Aufrechterhaltung der Katalysatoraktivität werden bevorzugt 0,01-0,15 Gew.-% Wasserstoff (bezogen auf den Reaktorfeed EDA+H₂) in den Reaktor gefahren.

Im bevorzugt kontinuierlichen Betrieb werden bei einer WHSV von 0,5-1,5 kg/kg*h (kg EDA pro kg Kat. pro Stunde) im Umsatzbereich von 15-30 % Selektivitäten (S) bezüglich DETA von bevorzugt ≥ 75 %, insbesondere 75-85 %, erreicht.

Als Nebenprodukte fallen bei dieser Umsetzung geringe Mengen an Piperazin (S_{PIP} im allgemeinen 8-13 %) und Triethylentetramin (S_{TETA} im allgemeinen 5-10 %) an.

Der Ammoniak- und gegebenenfalls optional Wasserstoff-haltige Reaktionsaustrag der separaten Umsetzung von EDA zu DETA wird in einer Ausführungsform des erfindungsgemäßen Verfahrens (Variante 1) mit dem Austrag des Reaktors 1 vereinigt und gemeinsam aufgearbeitet, also der Auftrennung des aus Reaktor 1 resultierenden Reaktionsaustrags, insbesondere der ersten Trennsequenz (Trennsequenz 1) der Auftrennung des aus Reaktor 1 resultierenden Reaktionsaustrags, zugeführt.

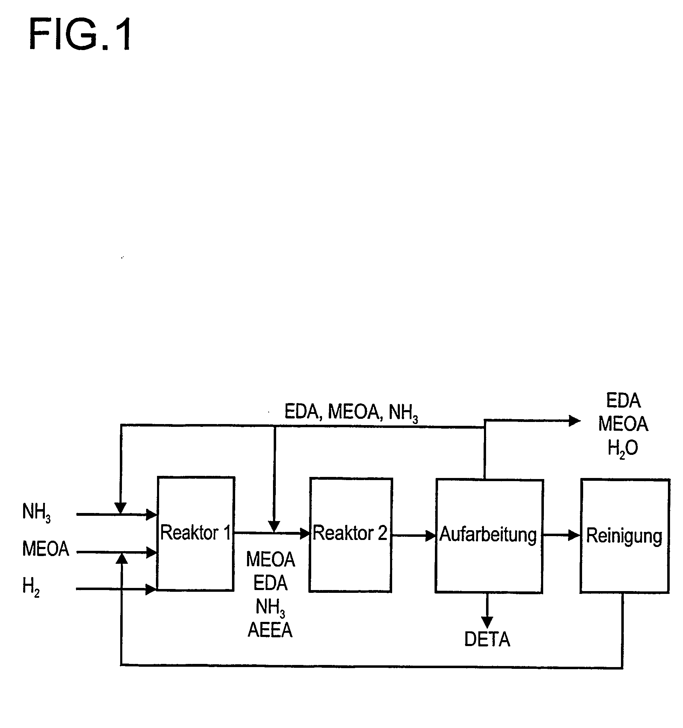

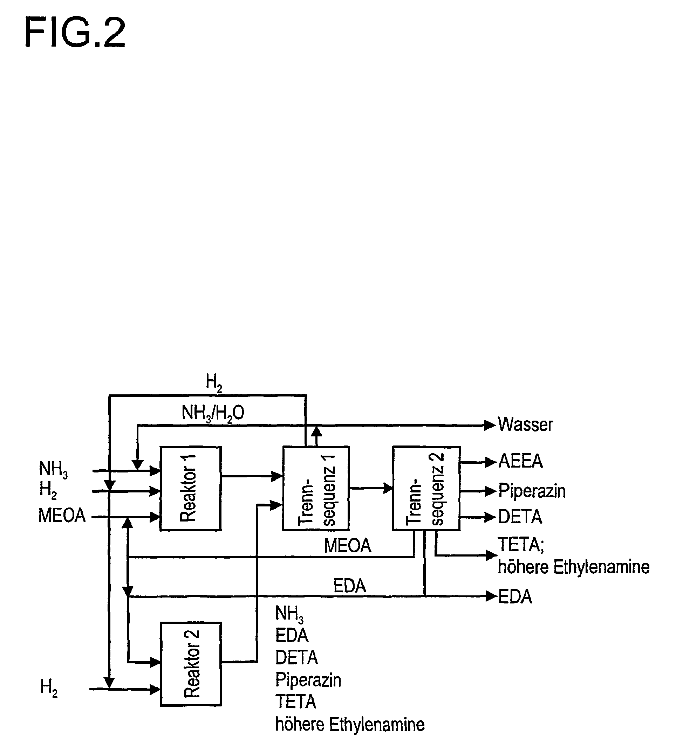

Ein Verfahrensschema dieser Variante 1 des erfindungsgemäßen Verfahrens befindet sich in Anlage 2 (Abbildung 2).

In einer weiteren Ausführungsform des erfindungsgemäßen Verfahrens (Variante 2) wird aus dem Reaktionsaustrag der separaten Umsetzung von EDA zu DETA zunächst Ammoniak und gegebenenfalls Wasserstoff abgetrennt (Trennsequenz 3) und der zurückbleibende Reaktionsaustrag, enthaltend Ethylenamine, dann der zweiten Trennsequenz (Trennsequenz 2) der Auftrennung des aus Reaktor 1 resultierenden Reaktionsaustrags zugeführt.

Ein Verfahrensschema dieser Variante 2 des erfindungsgemäßen Verfahrens befindet sich in Anlage 3 (Abbildung 3).

In einer weiteren speziellen Ausführungsform des erfindungsgemäßen Verfahrens (Variante 3, die eine spezielle Ausgestaltung von Variante 2 darstellt) wird die Umsetzung von Ethylendiamin zu Diethylentriamin unter Ammoniakabspaltung in einer Reaktionskolonne, bevorzugt kontinuierlich, durchgeführt (Reaktivdestillation).

Die Rückführung von nicht umgesetztem Ethylendiamin erfolgt dabei über den Rücklauf der Reaktionskolonne, Ammoniak und gegebenenfalls Wasserstoff werden über Kopf abgetrennt und jeweils optional in das Verfahren (in den Reaktor 1) zurückgeführt.

Ein Vorteil dieser Variante ist die kontinuierliche Abtrennung von Ammoniak aus dem Kondensationsgleichgewicht. Die Reaktionstemperatur wird dabei über den Kolonnendruck eingestellt.

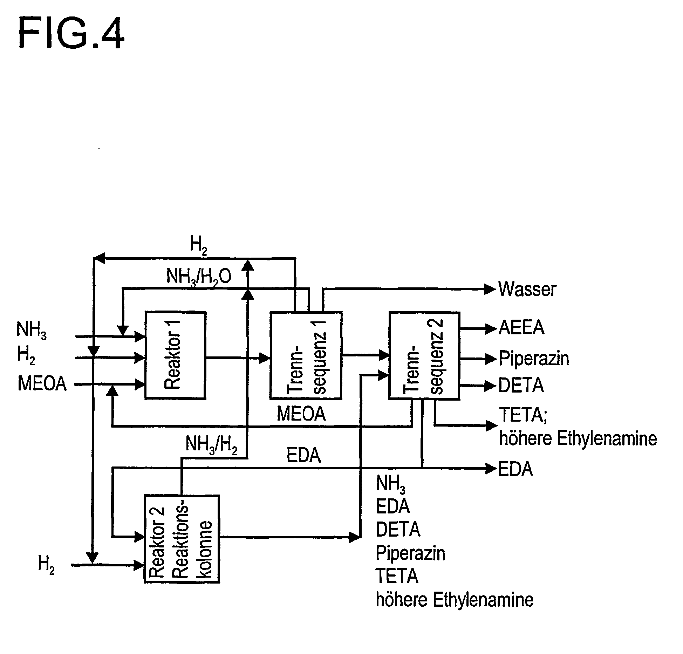

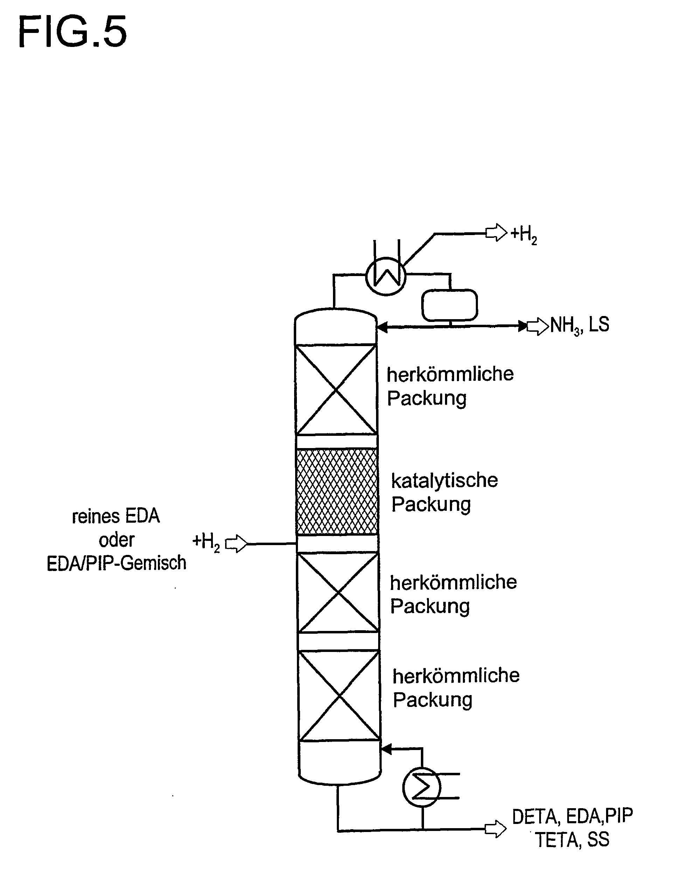

Ein Verfahrensschema dieser Variante 3 des erfindungsgemäßen Verfahrens befindet sich in Anlage 4 (Abbildung 4).

Die Auslegung der Reaktionskolonne (z.B. Zahl der Trennstufen in den Kolonnenabschnitten Verstärkungsteil, Abtriebsteil und Reaktionszone, Rücklaufverhältnis, etc.) kann durch den Fachmann nach ihm geläufigen Methoden vorgenommen werden.

Reaktionskolonnen sind dem Fachmann z.B. aus G. Kaibel et al., Chem.-Ing.-Tech. 50 (1978), Nr. 8, Seiten 586-592, und der dort zitierten Literatur und WO-A1-97/35834 bekannt.
Die auch als Reaktivdestillation bezeichneten Verfahren sind z.B. auch in dem Lehrbuch ,Reactive Distillation', edited by K. Sundmacher und A. Kienle, Verlag Wiley-VCH (2003), ausführlich beschrieben.

Die kontinuierliche Umsetzung von Ethylendiamin zu Ethylenaminen, insbesondere zu Diethylentriamin, unter Ammoniakabspaltung in einer Reaktionskolonne in Gegenwart eines Heterogenkatalysators ist Gegenstand einer parallelen deutschen Patentanmeldung von BASF AG mit gleichem Anmeldetag.

Ein beispielhaftes Verfahrensschema für eine einsetzbare Reaktionskolonne befindet sich in Anlage 5. Demgemäß wird reines EDA oder ein EDA/PIP-Gemisch zusammen mit Wasserstoff der Reaktionskolonne kontinuierlich unterhalb der katalytischen Packung zugeführt und ein Gemisch enthaltend DETA, unumgesetztes EDA, PIP, TETA und Schwersieder (SS, d.h. Komponenten mit einem Siedepunkt höher als DETA) über Sumpf erhalten. Ammoniak, Wasserstoff und Leichtsieder (LS, d.h. Komponenten mit einem Siedepunkt tiefer als DETA) werden über Kopf abgetrennt.

Der Absolutdruck in der Reaktionskolonne für die Umsetzung von EDA zu DETA wird im allgemeinen auf 1-20 bar, bevorzugt auf 5-10 bar, und die Temperatur in der katalytisch aktiven Zone (Reaktionszone) im allgemeinen auf 100-200°C, bevorzugt auf 140-160°C eingestellt.

Als katalytisch aktive Zone in der Reaktionskolonne wird ein heterogener Katalysator bevorzugt entweder lose in eine konventionelle Destillationspackung geschüttet oder ein Packungsmaterial mit katalytisch aktiver Oberfläche (Dünnschichtkatalyse) verwendet.

Als katalytisch aktives Material können sowohl Übergangsmetalle (z.B. Ni, Co, Cu, Ru, Re, Rh, Pd und/oder Pt) als auch zeolithische Beschichtungen oder Phosphatkatalysatoren eingesetzt werden.
Das oder die Metalle des Übergangsmetallkatalysators sind bevorzugt auf einem oxidischen Träger (z.B. Al₂O₃, TiO₂, ZrO₂, SiO₂) aufgebracht.

Die katalytisch aktive Zone besteht aus im allgemeinen 5-30, bevorzugt 10-20, theoretischen Trennstufen oberhalb des Kolonnenfeeds und die destillativen Zonen aus jeweils im allgemeinen 5-30, bevorzugt 10-20, theoretischen Trennstufen oberhalb und unterhalb der katalytisch aktiven Packung.

Das Kolonnen-Rücklauf : Feed - Gewichtsverhältnis beträgt im allgemeinen 0,5-10, bevorzugt 0,5-2.

Zur Aufrechterhaltung der Katalysatoraktivität wird, bevorzugt unterhalb der katalytischen Packung, bevorzugt Wasserstoff zugeführt. Die bevorzugte Menge dabei sind 0,01 bis 1 Gew.-% Wasserstoff bezogen auf die Feedmenge an EDA.

Freigesetzter Ammoniak und gegebenenfalls Wasserstoff wird über Kopf abgetrennt und die Reaktionsprodukte DETA, Piperazin und höhere Ethylenamine gemeinsam mit unumgesetztem Ethylendiamin über Sumpf.

Der EDA-Umsatz kann dabei über die Sumpftemperatur eingestellt werden.

Charakteristisch für das erfindungsgemäße Verfahren ist, dass durch die Integration der Kondensationsstufe EDA → DETA (Reaktor 2) in ein konventionelles Ethylenamineverfahren auf Basis Monoethanolamin beide Reaktorausträge gemeinsam aufgearbeitet werden.

Freigesetzter, bei der Aufarbeitung anfallender Ammoniak kann in die Aminierung von MEOA (Reaktor 1) zurückgefahren und Abgas-Wasserstoff, der gegebenenfalls in der Aminierung von MEOA anfällt, kann als Feed für den Kondensationsreaktor (Reaktor 2) verwendet werden.

Es wurde gefunden, dass die Vermischung der beiden Austräge aus Reaktor 1 und 2 vor der Aufarbeitung und die o.g. mögliche Verwertung von Ammoniak und Abgas-Wasserstoff keinen negativen Einfluss auf die Produktqualität der Ethylenamine, insbesondere von EDA und DETA, haben.

Im Vergleich zur direkten Weiterverarbeitung des Reaktionsaustrags aus Reaktor 1 gemäß EP-A2-197 611 werden über die selektive Konvertierung von EDA in dem Reaktor 2 erheblich höhere Selektivitäten bezüglich linearer Ethylenamine (EDA + DETA) erzielt.

### Beispiele

### Beispiel 1

Ein Gemisch aus Monoethanolamin, Ammoniak und Wasserstoff (Molverhältnis MEOA : NH₃: H₂ = 1 : 8: 0,14) wurde mit einer WHSV von 0,4 kg/kg/h (kg MEOA pro kg Katalysator pro Stunde) bei einer Temperatur von 170°C und einem Druck von 200 bar kontinuierlich in einen Rohreaktor (Reaktor 1) gefahren, der mit einem Katalysator, bestehend aus 4 Gew.-% Kupfer, 6 Gew.-% Kobalt und 8 Gew.-% Nickel (jeweils bezogen auf den geträgerten Katalysator) auf einem Aluminiumoxidträger, gefüllt war. Als Austrag erhielt man ein Gemisch bestehend aus 65,7 Gew.-% Ammoniak, 4,4 Gew.-% Wasser, 15,5 Gew.-% MEOA, 10,9 Gew.-% EDA, 1,3 Gew.-% DETA, 0,9 Gew.-% AEEA, 0,74 Gew.-% Piperazin und 0,56 Gew.-% TETA, AEP, HEP + höhere Ethylenamine. (höhere Ethylenamine = Ethylenamine mit einem (bei gleichem Druck) höheren Siedepunkt als TETA).

In einen zweiten Reaktor (Reaktor 2), der ebenfalls mit einem Katalysator, bestehend aus 4 Gew.-% Kupfer, 6 Gew.-% Kobalt und 8 Gew.-% Nickel (jeweils bezogen auf den geträgerten Katalysator) auf einem Aluminiumoxidträger, gefüllt war, wurde mit einer WHSV von 0,7 kg/kg/h (kg EDA pro kg Katalysator pro Stunde) ein Gemisch aus Ethylendiamin und Wasserstoff (Molverhältnis 50 : 1) gefahren. Der Reaktionsdruck wurde dabei auf 30 bar und die Reaktortemperatur auf 150°C eingestellt. Als Austrag erhielt man ein Gemisch bestehend aus 5,1 Gew.-% Ammoniak, 69,9 Gew.-% Ethylendiamin, 19,3 Gew.-% Diethylentriamin, 2,6 Gew.-% Piperazin und 3,1 Gew.-% TETA, AEP, HEP + höhere Ethylenamine.

Die Austräge der beiden Reaktoren werden vereinigt und per mehrstufiger kontinuierlicher Destillation in die einzelnen Komponenten aufgetrennt. Bei 80 %iger Rückführung von dabei anfallendem Ethylendiamin in den Reaktor 2 erhält man ein Produktgemisch bestehend aus 28 Gew.-%_Ethylendiamin, 24 Gew.-% Wasser, 28 Gew.-% Diethylentriamin, 8 Gew.-% Piperazin, 6 Gew.-% Aminoethylethanolamin und 6 Gew.-% TE-TA, AEP, HEP + höhere Ethylenamine.

Vor der Ammoniakabtrennung anfallender Niederdruckwasserstoff wird zur Aufrechterhaltung der Katalysatoraktivität in den Reaktor 2 gefahren.

Freigesetzter Ammoniak aus der Kondensation von Ethylendiamin zu Diethylentriamin wird in die Aminierung von Monoethanolamin (in Reaktor 1) zurückgeführt.

### Beispiel 2

Ein Gemisch aus Monoethanolamin, Ammoniak und Wasserstoff (Molverhältnis MEOA : NH₃ : H₂ = 1 : 10 : 0,14) wurde mit einer WHSV 0,4 kg/kg/h (kg MEOA pro kg Katalysator pro Stunde) bei einer Temperatur von 170°C und einem Druck von 200 bar kontinuierlich in einen Rohreaktor gefahren, der mit einem Cu/Co/Ni-Katalysator wie in Beispiel 1 gefüllt war. Als Austrag erhielt man ein Gemisch bestehend aus 70,7 Gew.-% Ammoniak, 3,7 Gew.-% Wasser, 13,2 Gew.-% MEOA, 9,5 Gew.% EDA, 1,1 Gew.-% DETA, 0,68 Gew.-% AEEA, 0,56 Gew.-% Piperazin und 0,56 Gew.-% TETA, AEP, HEP + höhere Ethylenamine.

In einen zweiten Reaktor (Reaktor 2), der ebenfalls mit einem Cu/Co/Ni-Katalysator wie in Beispiel 1 gefüllt war, wurde mit einer WHSV von 1,1 kg/kg/h (kg EDA pro kg Katalysator pro Stunde) ein Gemisch aus Ethylendiamin und Wasserstoff (Molverhältnis 50 : 1) gefahren. Der Reaktionsdruck wurde dabei auf 30 bar und die Reaktortemperatur auf 160°C eingestellt. Als Austrag erhielt man eine Gemisch bestehend aus 4,2 Gew.-% Ammoniak, 75,0 Gew.-% Ethylendiamin, 16,7 Gew.-% Diethylentriamin, 2,6 Gew.-% Piperazin und 1,5 Gew.% TETA, AEP, HEP + höhere Ethylenamine.

Die Austräge der beiden Reaktoren werden vereinigt und per kontinuierlicher Destillation in die einzelnen Komponenten aufgetrennt. Bei 60 %iger Rückführung von dabei anfallendem Ethylendiamin in den Reaktor 2 erhält man ein Produktgemisch bestehend aus 37 Gew.-% Ethylendiamin, 24 Gew.-% Wasser, 23,4 Gew.-% Diethylentriamin, 6 Gew.-% Piperazin, 4,4 Gew.-% Aminoethylethanolamin und 5,2 Gew.-% TETA, AEP, HEP + höhere Ethylenamine.

Vor der Ammoniakabtrennung anfallender Niederdruckwasserstoff wird zur Aufrechterhaltung der Katalysatoraktivität in den Reaktor 2 gefahren.

Freigesetzter Ammoniak aus der Kondensation von Ethylendiamin zu Diethylentriamin wird in die Aminierung von Monoethanolamin (in Reaktor 1) zurückgeführt.

## Patentansprüche

1. Verfahren zur Herstellung von Ethylenaminen durch Umsetzung von Monoethanolamin (MEOA) mit Ammoniak in Gegenwart eines Katalysators in einem Reaktor (1) und Auftrennung des resultierenden Reaktionsaustrags, **dadurch gekennzeichnet, dass** bei der Auftrennung erhaltenes Ethylendiamin (EDA) in einem separaten Reaktor (2) in Gegenwart eines Katalysators zu Diethylentriamin (DETA) umgesetzt und der resultierende Reaktionsaustrag der Auftrennung des aus Reaktor 1 resultierenden Reaktionsaustrags zugeführt wird.

2. Verfahren zur Herstellung von Ethylenaminen nach Anspruch 1, wobei es sich bei den Ethylenaminen um EDA, DETA, Aminoethylethanolamin (AEEA), Piperazin (PIP) und/oder Triethylentetramin (TETA) handelt.

3. Verfahren zur Herstellung von Ethylenaminen nach den Ansprüchen 1 oder 2, wobei der Anteil an DETA größer 20 Gew.-% beträgt.

4. Verfahren nach einem der vorhergehenden Ansprüche, **dadurch gekennzeichnet, dass** die Umsetzung in Reaktor 1 in Gegenwart eines Übergangsmetallkatalysators oder eines Zeoliths durchgeführt wird.

5. Verfahren nach dem vorhergehenden Anspruch, **dadurch gekennzeichnet, dass** die übergangsmetallkatalysierte Umsetzung in Reaktor 1 in Gegenwart von Wasserstoff durchgeführt wird.

6. Verfahren nach einem der vorhergehenden Ansprüche, **dadurch gekennzeichnet, dass** die Auftrennung des aus Reaktor 1 resultierenden Reaktionsaustrags durch mehrstufige Destillation erfolgt.

7. Verfahren nach einem der vorhergehenden Ansprüche, **dadurch gekennzeichnet, dass** die Auftrennung des aus Reaktor 1 resultierenden Reaktionsaustrags in zwei Trennsequenzen durch mehrstufige Destillation erfolgt, wobei in der ersten Trennsequenz zunächst Ammoniak, Wasser und gegebenenfalls vorhandener Wasserstoff abgetrennt werden und in der zweiten Trennsequenz eine Auftrennung in EDA, PIP, MEOA, DETA, AEP, HEP, AEEA, TETA und höhere Ethylenamine erfolgt.

8. Verfahren nach einem der vorhergehenden Ansprüche, **dadurch gekennzeichnet, dass** die Umsetzung in Reaktor 2 in Gegenwart eines Übergangsmetallkatalysators, eines Zeoliths oder eines Phosphatkatalysators durchgeführt wird.

9. Verfahren nach dem vorhergehenden Anspruch, **dadurch gekennzeichnet, dass** die übergangsmetallkatalysierte Umsetzung in Reaktor 2 in Gegenwart von Wasserstoff durchgeführt wird.

10. Verfahren nach einem der Ansprüche 7 bis 9, **dadurch gekennzeichnet, dass** der aus Reaktor 2 resultierende Reaktionsaustrag, enthaltend Ammoniak und DETA, der ersten Trennsequenz der Auftrennung des aus Reaktor 1 resultierenden Reaktionsaustrags zugeführt wird.

11. Verfahren nach einem der Ansprüche 7 bis 9, **dadurch gekennzeichnet, dass** aus dem aus Reaktor 2 resultierenden Reaktionsaustrag Ammoniak und gegebenenfalls Wasserstoff abgetrennt wird (Trennsequenz 3) und der Reaktionsaustrag dann der zweiten Trennsequenz der Auftrennung des aus Reaktor 1 resultierenden Reaktionsaustrags zugeführt wird.

12. Verfahren nach dem vorhergehenden Anspruch, **dadurch gekennzeichnet, dass** die Umsetzung des EDAs zu DETA und die Abtrennung des Ammoniaks in einer Reaktionskolonne durchgeführt wird.

13. Verfahren nach einem der vorhergehenden Ansprüche, **dadurch gekennzeichnet, dass** bei der Auftrennung des aus Reaktor 1 resultierenden Reaktionsaustrags anfallender Ammoniak in den Reaktor 1 zurückgeführt wird.

14. Verfahren nach einem der Ansprüche 11 bis 12, **dadurch gekennzeichnet, dass** der aus dem resultierenden Reaktionsaustrag des Reaktors 2 abgetrennte Ammoniak bzw. der aus der Reaktionskolonne abgetrennte Ammoniak in den Reaktor 1 zurückgeführt wird.

## Claims

1. A process for the preparation of ethyleneamines by reacting monoethanolamine (MEOA) with ammonia in the presence of a catalyst in a reactor (1) and separating the resulting reaction product, which comprises reacting ethylenediamine (EDA) obtained during the separation in a separate reactor (2) in the presence of a catalyst to give diethylenetriamine (DETA), and the resulting reaction product is passed to the separation of the reaction product resulting from reactor 1.

2. The process for the preparation of ethyleneamines according to claim 1, where the ethyleneamines are EDA, DETA, aminoethylethanolamine (AEEA), piperazine (PIP) and/or triethylenetetramine (TETA).

3. The process for the preparation of ethyleneamines according to claims 1 or 2, where the proportion of DETA is greater than 20% by weight.

4. The process according to any of the preceding claims, wherein the reaction in reactor 1 is carried out in the presence of a transition metal catalyst or a zeolite.

5. The process according to the preceding claim wherein the transition-metal-catalyzed reaction in reactor 1 is carried out in the presence of hydrogen.

6. The process according to any of the preceding claims, wherein the separation of the reaction product resulting from reactor 1 takes place by multistage distillation.

7. The process according to any of the preceding claims, wherein the separation of the reaction product resulting from reactor 1 takes place in two separation sequences by multistage distillation, where in the first separation sequence firstly ammonia, water and if appropriate hydrogen present are separated off, and in the second separation sequence a separation into EDA, PIP, MEOA, DETA, AEP, HEP, AEEA, TETA and higher ethyleneamines takes place.

8. The process according to any of the preceding claims, wherein the reaction in reactor 2 is carried out in the presence of a transition metal catalyst, a zeolite or a phosphate catalyst.

9. The process according to the preceding claim, wherein the transition-metal-catalyzed reaction in reactor 2 is carried out in the presence of hydrogen.

10. The process according to any of claims 7 to 9, wherein the reaction product resulting from reactor 2, comprising ammonia and DETA, is passed to the first separation sequence of the separation of the reaction product resulting from reactor 1.

11. The process according to any of claims 7 to 9, wherein ammonia and if appropriate hydrogen is separated off from the reaction product resulting from reactor 2 (separation sequence 3) and the reaction product is then passed to the second separation sequence of the separation of the reaction product resulting from reactor 1.

12. The process according to the preceding claim, wherein the reaction of the EDA to give DETA and the removal of the ammonia is carried out in a reaction column.

13. The process according to any of the preceding claims, wherein ammonia that is produced during the separation of the reaction product resulting from reactor 1 is returned to reactor 1.

14. The process according to either of claims 11 and 12, wherein the ammonia separated off from the resulting reaction product of reactor 2 or the ammonia separated off from the reaction column is returned to reactor 1.

## Revendications

1. Procédé de préparation d'éthylèneamines par réaction de monoéthanolamine (MEOA) avec de l'ammoniac en présence d'un catalyseur dans un réacteur (1) et séparation de l'effluent réactionnel résultant, **caractérisé en ce qu'**on transforme de l'éthylène-diamine (EDA) obtenue au cours de la séparation dans un réacteur séparé (2) en présence d'un catalyseur en de la diéthylènetriamine (DETA) et on amène l'effluent réactionnel résultant à la séparation de l'effluent réactionnel résultant du réacteur (1).

2. Procédé de préparation d'éthylèneamines suivant la revendication 1, dans lequel, pour ce qui concerne les éthylèneamines, il s'agit d'EDA, de DETA, d'aminoéthyléthanolamine (AEEA), de pipérazine (PIP) et/ou de triéthylènetétramine (TETA).

3. Procédé de préparation d'éthylèneamines suivant les revendications 1 ou 2, dans lequel la fraction de DETA est supérieure à 20 % en poids.

4. Procédé suivant l'une des revendications précédentes, **caractérisé en ce que** la réaction dans le réacteur (1) est effectuée en présence d'un catalyseur à base de métal de transition ou d'une zéolite.

5. Procédé suivant la revendication précédente, **caractérisé en ce que** la réaction catalysée par un métal de transition est effectuée dans le réacteur (1) en présence d'hydrogène.

6. Procédé suivant l'une des revendications précédentes, **caractérisé en ce que** la séparation de l'effluent réactionnel résultant du réacteur (1) a lieu par distillation en plusieurs étapes.

7. Procédé suivant l'une des revendications précédentes, **caractérisé en ce que** la séparation de l'effluent réactionnel résultant du réacteur (1) a lieu en deux séquences séparées par distillation en plusieurs étapes, l'ammoniac, l'eau et éventuellement l'hydrogène présent étant tout d'abord isolés dans la première séquence de séparation et une séparation en EDA, PIP, MEOA, DETA, AEP, HEP, AEEA, TETA et des éthylèneamines supérieures ayant lieu dans la deuxième séquence de séparation.

8. Procédé suivant l'une des revendications précédentes, **caractérisé en ce que** la réaction dans le réacteur (2) est effectuée en présence d'un catalyseur à base d'un métal de transition, d'une zéolite ou d'un catalyseur à base de phosphate.

9. Procédé suivant la revendication précédente, **caractérisé en ce que** la réaction catalysée par un métal de transition est effectuée dans le réacteur (2) en présence d'hydrogène.

10. Procédé suivant l'une des revendications 7 à 9, **caractérisé en ce que** l'effluent réactionnel résultant du réacteur (2), contenant de l'ammoniac et de la DETA, est amené à la première séquence de séparation de la séparation de l'effluent réactionnel résultant du réacteur (1).

11. Procédé suivant l'une des revendications 7 à 9, **caractérisé en ce que**, à partir de l'effluent réactionnel résultant du réacteur (2), on isole de l'ammoniac et éventuellement de l'hydrogène (séquence de séparation 3) et l'effluent réactionnel est alors amené à la deuxième séquence de séparation de la séparation de l'effluent réactionnel résultant du réacteur (1).

12. Procédé suivant la revendication précédente, **caractérisé en ce que** la transformation du EDA en DETA et l'isolement de l'ammoniac sont effectués dans une colonne réactionnelle.

13. Procédé suivant l'une des revendications précédentes, **caractérisé en ce que**, lors de la séparation de l'effluent réactionnel résultant du réacteur (1), l'ammoniac obtenu est recyclé dans le réacteur (1).

14. Procédé suivant l'une des revendications 11 et 12, **caractérisé en ce que** l'ammoniac isolé à partir de l'effluent réactionnel résultant du réacteur (2) et respectivement l'ammoniac isolé de la colonne réactionnelle sont recyclés dans le réacteur (1).
